# EUROPEAN PATENT APPLICATION

(11) **EP 1 922 992 A1**
(43) Date of publication of application: **21.05.2008**
(21) Application number: 06767904.3
(22) Date of filing: 05.07.2006
(51) Int. Cl.: A61B 5/022

(54) **BLOOD PRESSURE METER**

(30) Priority: 09.09.2005 JP 2005263000
(71) Applicant: CITIZEN HOLDINGS CO., LTD., Nishitokyo-shi, Tokyo 188-8511 (JP)
(72) Inventor: TAKAHASHI, Kazunari, Nishitokyo-shi, Tokyo 188-8511 (JP); SUZUKI, Takako, Nishitokyo-shi, Tokyo 188-8511 (JP)
(74) Representative: Liebetanz, Michael
(86) International application number: PCT/JP2006/313398
(87) International publication number: WO 2007/029408

(57) **Abstract**

In a blood pressure meter (1) including: a body which is connected through a tube with a cuff and which has a display section (14) which displays a blood pressure value determined by a control section (6) based on a variation in a pressure measurement value by a pressure sensor (5) in the cuff; a spherical hand pump (3) which is attached to the body (2) and which supplies air by a compressing operation to the cuff to inflate the cuff; and a grasp section (16) which is extended from the body (2) along the outer surface of the pump (3) in the direction of the axis of the hand pump (3) and which is capable of being grasped with the pump (3), the display section (14) being placed at the body (2) so that a blood pressure value is visible from a direction substantially normal to the axis of the pump (3), the center line in the width direction of the grasp section (16) is placed at a position at 45° about the axis line of the pump (3) relative to the display (14).

## Description

### Technical Field

The present invention relates to a blood pressure meter in which a spherical hand pump which inflates a cuff is integrally provided to a body having a display section which displays a blood pressure value.

### Background Art

A known blood pressure meter includes a body connected through a tube with a cuff which is detachably disposed to an arm of a measurement target person, and a spherical hand pump which is attached to the body and supplies air to the cuff by a compressing operation to inflate the cuff.

The body has a built-in pressure sensor which measures a pressure in the cuff, and a built-in control section which determines a blood pressure value based on a variation in the measured value. Further, on an outer surface of the body, a display section such as a liquid crystal panel which displays the blood pressure value determined by the control section is arranged in such a manner that the displayed blood pressure value can be seen from a direction substantially perpendicular to an axis line of the hand pump. Furthermore, the body is provided with a grasp section which facilitates the compressing operation of the hand pump.

This grasp section is extended along the outer surface of the hand pump from the body in the axial direction of the hand pump, and arranged in the same phase as that of the display section [a position at 0° about the axis line of the hand pump with respect to the display section (see, e.g., Patent Document 1 and Patent Document 2)] or at a side position of the display section [a position at 90° about the axis line of the hand pump with respect to the display section (see, e.g., Patent Document 3)] in a conventional example.
Patent Document 1: PCT National Publication No. 2004-515257, FIG. 2
Patent Document 2: Design Registration Publication No. 1155988
Patent Document 3: Japanese Examined Utility Model Publication No. 10566-1983

Meanwhile, since the compressing operation of the hand pump is mainly performed by a pressure of fingers, it is preferable to grasp the grasp section in a direction of a depressed part of a palm (a direction along which an intellectual line extends) from a part between a thumb and an index finger as far as possible so that the fingers can freely move without coming into contact with the grasp section.

However, when the grasp section is arranged to have the same phase as the display section like Patent Document 1 and Patent Document 2, grasping the grasp section in the direction of the depressed part of the palm prevents the display section from facing a front side with respect to an operator, and a blood pressure value displayed in the display section is hard to see. Moreover, since the grasp section is arranged on a lateral side of the display section in Patent Document 3, grasping the grasp section in the direction of the depressed part of the palm prevents the display section from facing the front side with respect to the operator for the same reason, and the blood pressure value displayed in the display section is hard to see.

That is, in Patent Documents 1 to 3, in case of grasping the grasp section together with the hand pump to compress the hand pump, when the display section is turned to face the front side with respect to the operator so that the blood pressure value displayed in the display section can be readily seen, the fingers come into contact with the grasp section, which makes it difficult to compress the hand pump. On the other hand, when the fingers are prevented from coming into contact with the grasp section to facilitate the compressing operation of the hand pump, there is a problem that the display section does not face the front side with respect to the operator and the blood pressure value displayed in the display section is hard to see.

In order to eliminate such an inconvenience, it is an object of the present invention to provide a blood pressure meter which turns a display section to a substantially front side with respect to a user so that a blood pressure value displayed in the display section can be easily seen when grasping a grasp section together with a hand pump to compress the hand pump, and prevents fingers from coming into contact with the grasp section to facilitate the compressing operation of the hand pump.

### Disclosure of the Invention

To achieve this object, according to the present invention, there is provided a blood pressure meter comprising a body which is connected through a tube with a cuff detachably disposed to an arm of a measurement target person, has a pressure sensor which measures a pressure in the cuff, has a control section which determines a blood pressure value based on a fluctuation in the measured value, and has a display section which displays the determined blood pressure value; a spherical hand pump which is attached to the body and supplies air to the cuff by a compressing operation to inflate the cuff; and a grasp section which is extended along an outer surface of the hand pump from the body in an axial direction of the hand pump and configured to be grasped together with the hand pump, the display section being arranged in the body so that the blood pressure value is visible from a direction substantially perpendicular to an axis line of the hand pump, wherein a central line of the grasp section in a width direction is arranged in a range of not less than 10° to less than 80° around the axis line of the hand pump with respect to the display section.

When this configuration is adopted, the grasp section can be grasped in a direction of a depressed part of a palm (a direction along which an intellectual line extends) from a part between a thumb and an index finger in a state where the display section faces a substantially front side with respect to an operator of the hand pump. Therefore, the fingers can freely move without coming into contact with the grasp section, and a movement of the hand pump with respect to the body can be suppressed. As a result, the compressing operation of the hand pump can be efficiently repeated, and the display section can be seen from the substantially front side during an operation to readily confirm a blood pressure value.

Additionally, according to the present invention, it is preferable that the central line of the grasp section in the width direction is arranged at a position at 45° around the axis line of the hand pump with respect to the display section.

When such a configuration is adopted, since the grasp section can be grasped in the direction of the depressed part of the palm (the direction along which the intellectual line extends) from the part between the thumb and the index finger in a state where the display section faces the front side with respect to the operator of the hand pump, the display section can be seen from the front side during an operation so that a blood pressure value displayed in the display section can be readily seen.

Further, in the present invention, it is preferable that a width dimension of the grasp section is less than 90° around the axis of the hand pump.

When such a configuration is adopted, an area of the grasp section covering an outer surface of the hand pump can be reduced, and a grasped area of the hand pump can be increased when the hand pump is grasped together with the grasp section by a hand. As a result, a pump reduction ratio when compressing the hand pump can be earned, thereby efficiently compressing the hand pump.

Furthermore, in the present invention, it is preferable that an extended end of the grasp section from the body is arranged at a position which exceeds 1/2 of a length of the hand pump in the axial direction and is less than 4/5 of the same.

When such a configuration is adopted, the area of the grasp section covering the outer surface of the hand pump can be reduced, and the grasped area of the hand pump can be increased when grasping the hand pump together with the grasp section by a hand. As a result, a pump reduction ratio when compressing the hand pump can be earned, thereby efficiently compressing the hand pump.

Moreover, in the compressing operation of the hand pump, the hand pump can be prevented from bending with an extended end of the grasp section being used as a supporting point.

Additionally, in the present invention, it is preferable that the extended end of the grasp section from the body is arranged on a side where it is apart from the body beyond a maximum diameter of the hand pump.

When such a configuration is adopted, in the compressing operation of the hand pump, the hand pump can be assuredly prevented from bending with an extended end of the grasp section being used as a supporting point.

Further, in the present invention, it is preferable that the grasp section is arranged to form a gap between itself and an outer surface of the hand pump along an extending direction from the body, and the extended end is in contact with the hand pump.

When such a configuration is adopted, a compression stroke can be increased by an amount corresponding to the gap in the compressing operation of the hand pump. As a result, the reduction ratio when compressing the hand pump can be further earned. Furthermore, since the extended end of the grasp section is in contact with the hand pump, operability of the compressing operation of the hand pump is not limited.

Moreover, in the present invention, it is preferable that the grasp section is arranged to be rotatable around the axis line of the hand pump with respect to the body, and the blood pressure meter includes restricting means for positioning and restricting the grasp section at a predetermined rotation angle.

When such a configuration is adopted, irrespective of right handedness and left handedness of an operator, a central line of the grasp section in the width direction can be arranged in a range of not less than 10° to less than 80° about the axis line of the hand pump with respect to the display section.

According to the present invention, when the grasp section is grasped together with the hand pump to compress the hand pump, the display section can be turned to the substantially front side with respect to a user so that a blood pressure value displayed in the display section can be easily viewed, and the fingers can be prevented from coming into contact with the grasp section to facilitate the compressing operation of the hand pump.

### Brief Description of the Drawings

FIG. 1 is a front view of a blood pressure meter as an embodiment according to the present invention;
FIG. 2 is a cross-sectional view taken along a line A-A in FIG. 1;
FIG. 3 is a view for explaining an arrangement range of a grasp section seen from a direction indicated by an arrow B in FIG. 1;
FIG. 4 is a view for explaining a preferable range of a width dimension of the grasp section seen from the direction indicated by the arrow B in FIG. 1;
FIG. 5 is a view for explaining a positional relationship between a maximum diameter of a hand pump and an extended end of the grasp section;
FIG. 6 is a view for explaining an effect of increasing a compression stroke of the hand pump based on a gap between the hand pump and the grasp section; and
FIG. 7 is a partially cutaway front view of a blood pressure meter according to another embodiment of the present invention.

### Best Mode for Carrying out the Invention

An embodiment according to the present invention will now be explained hereinafter with reference to the drawings.

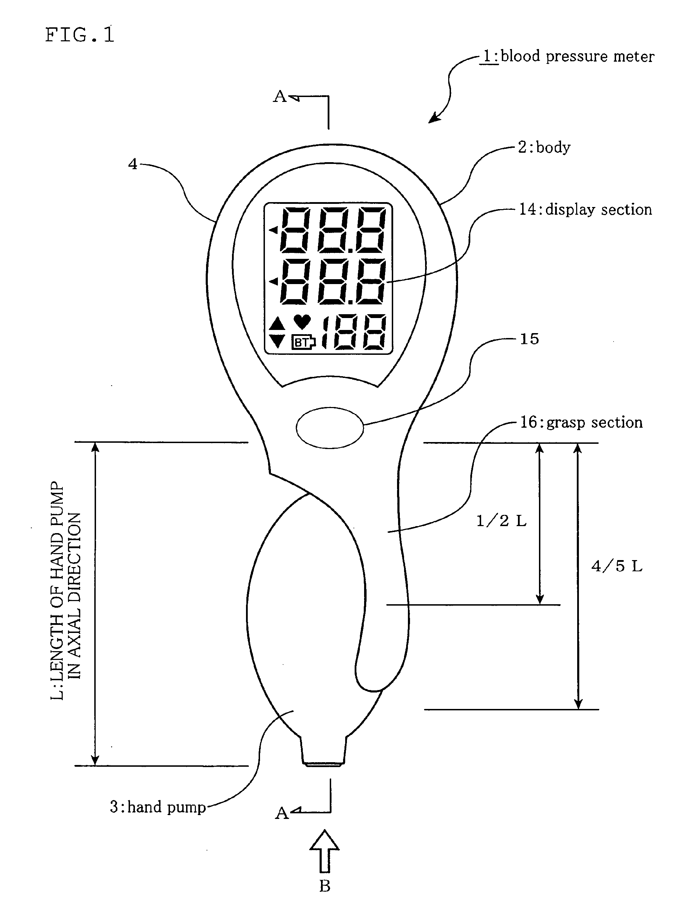

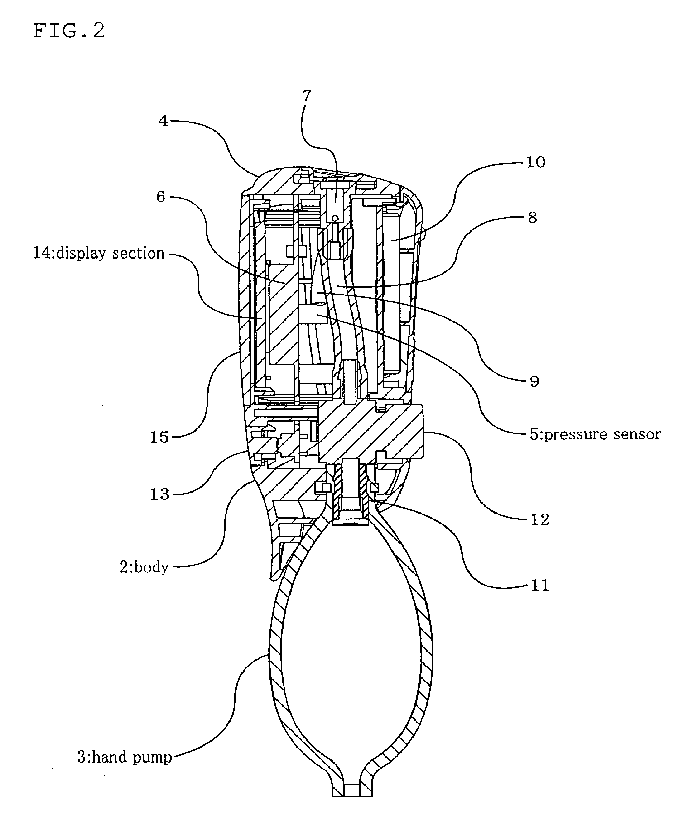

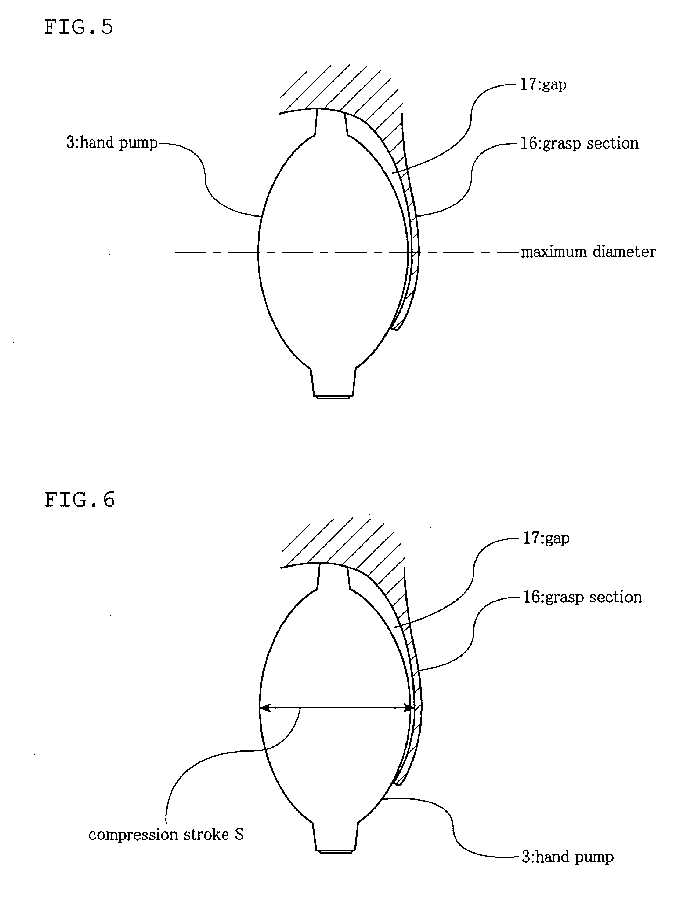

FIG. 1 is a front view of a blood pressure as an embodiment according to the present invention, FIG. 2 is a cross-sectional view taken along a line A-A in FIG. 1, FIGS. 3 and 4 are views seen from the direction indicated by the arrow B in FIG. 1, FIG. 5 is a view for explaining a positional relationship between a maximum diameter of a hand pump and an extended end of a grasp section, and FIG. 6 is a view for explaining an effect of increasing a compression stroke of the hand pump based on a gap between the hand pump and the grasp section.

A blood pressure meter 1 according to an embodiment of the present invention includes a body 2 connected through a tube with a cuff detachably disposed to an arm of a measurement target person (both the members are not shown) and a hand pump 3 formed of, e.g., a rubber ball which is attached to a lower portion of the body 2 and supplies air to the cuff by a compressing operation to inflate the cuff.

As shown in FIG. 2, the body 2 has a built-in pressure sensor 5 which measures a pressure in the cuff and a substrate 6 on which a control section determining a blood pressure value based on a variation in a measured value is mounted in a case 4 made of, e.g., a synthetic resin. A cuff connector 7 connected with the tube interposed between the body 2 and the cuff is provided at an upper portion of the body 2.

It is to be noted that reference numeral 8 denotes a tube forming an air flow path between the inside of the hand pump 3 and the cuff connector 7; 9, a tube forming an air flow path between the pressure sensor 5 and the cuff connector 7; 10, a power supply battery; 11, a constant-speed exhaust valve arranged at an end of the tube 8 on the hand pump 3 side; 12, a forcible exhaust button provided on a rear surface side of the body 2; and 13, a power supply switch provided on a front surface side of the body 2.

Additionally, a display section 14, e.g., a liquid crystal display panel which displays a blood pressure value (a systolic blood pressure value and a diastolic blood pressure value) determined by the control section mounted on the substrate 6 is arranged at a position above the power supply switch 13. In the display section 14, a displayed blood pressure value is visible through a windshield plate 15 from a direction substantially perpendicular to an axis line of the hand pump 3.

Further, a grasp section 16 is provided to the body 2 to facilitate a compressing operation and prevent the body 2 from falling when the hand pump 3 is grasped by a hand to perform the compressing operation. The grasp section 16 is extended along an outer surface of the hand pump 3 from the body 2 in an axial direction of the hand pump 3, and an extended end thereof is formed into an arc shape.

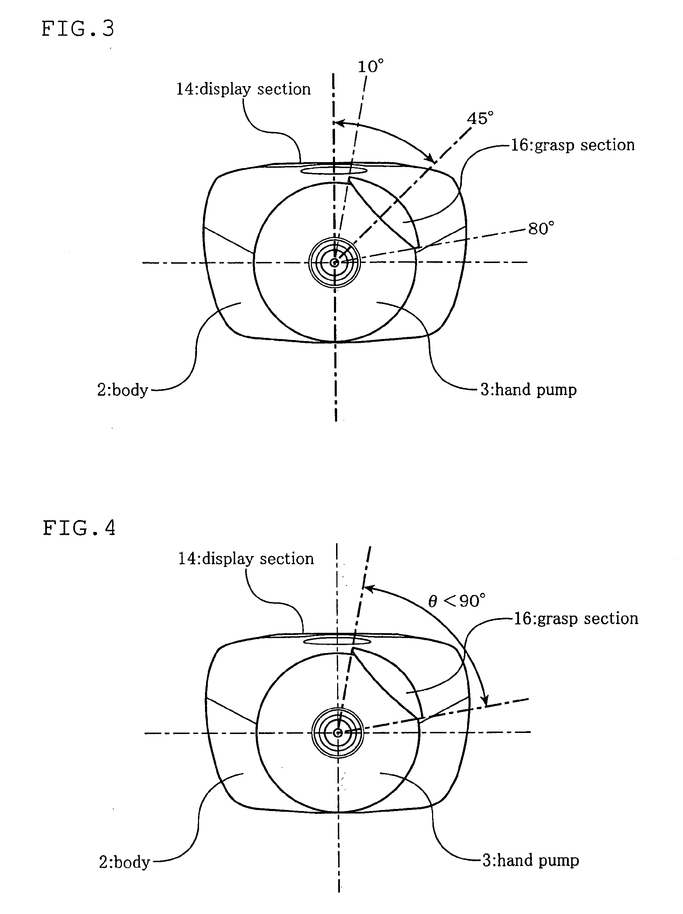

Furthermore, in this embodiment, as shown in FIG. 3, a central line of the grasp section 16 in a width direction is arranged at a position at 45° about the axis line of the hand pump 3 with respect to the display section 14. As a result, the grasp section 16 can be grasped in a direction of a depressed part of a palm (a direction along which an intellectual line extends) from a part between a thumb and an index finger in a state where the display section 14 is turned to a front side with respect to an operator of the hand pump 3.

Therefore, fingers can be freely moved without coming into contact with the grasp section 16, and a movement of the hand pump 3 with respect to the body 2 can be suppressed.

It is to be noted that the central line of the grasp section 16 in the width direction is not restricted to 45° around the axis line of the hand pump 3 with respect to the display section 14, and it can be arranged in a range of not less than 10° to less than 80°. When the central line of the grasp section 16 in the width direction is arranged in the range of not less than 10° to less than 80° around the axis line of the hand pump 3 with respect to the display section 14, the grasp section 16 can be grasped from the part between the thumb and the index finger in the direction of the depressed part of the palm (the direction along which the intellectual line extends) in a state where the display section 14 faces the substantially front side with respect to the operator of the hand pump 3.

In particular, when the central line of the grasp section 16 in the width direction is arranged in a range of 45°±10° around the axis line of the hand pump 3 with respect to the display section 14, the most excellent state of operability of the hand pump can be maintained in a state where the display section can be readily seen.

Moreover, as shown in FIG. 4, since a width dimension of the grasp section 16 is less than 90° about the axis of the hand pump 3, an area of the grasp section 16 covering an outer surface of the hand pump 3 can be reduced, and a grasped area of the hand pump 3 can be increased when the hand pump 3 is grasped together with the grasp section 16 by a hand. As a result, a pump reduction ratio when compressing the hand pump 3 can be earned, thereby efficiently compressing the hand pump 3.

Additionally, the extended end of the grasp section 16 from the body 2 is arranged at a position which exceeds 1/2 of a length L of the hand pump 3 in the axial direction and is less than 4/5 of the same as depicted in FIG. 1 on a side where it is apart from the body 2 beyond a maximum diameter of the hand pump 3 as shown in FIG. 5, and the area of the grasping section 16 covering the outer surface of the hand pump 3 is reduced, thus increasing the grasped area of the hand pump 3 when grasping the hand pump 3 together with the grasp section 16 by a hand.

As a result, the pump reduction ratio when compressing the hand pump 3 can be earned, the hand pump 3 can be further efficiently compressed, and the hand pump 3 can be assuredly prevented from bending with the extended end of the grasp section 16 being used as a supporting point when compressing the hand pump 3.

Further, as shown in FIG. 6, since the grasp section 16 is arranged to form a gap 17 between itself and the outer surface of the hand pump 3 along an extending direction from the body 2 and the extended end is in contact with the outer surface of the hand pump 3, operability of the compressing operation of the hand pump 3 is not limited, and a compression stroke S in the compressing operation of the hand pump 3 can be increased by an amount corresponding to the gap 17.

As a result, the reduction ratio when compressing the hand pump 3 can be further increased, and the hand pump 3 can be more efficiently compressed.

As explained above, in this embodiment, since the central line of the grasp section 16 in the width direction is arranged at the position at 45° about the axis line of the hand pump 3 with respect to the display section 14, the grasp section 16 can be grasped from the part between the thumb and the index finger in the direction of the depressed part of the palm (the direction along which the intellectual line extends) in a state where the display section 14 faces the front side with respect to the operator of the hand pump 3.

Therefore, the fingers can be freely moved without bringing the fingers into contact with the grasp section 16, thus suppressing a movement of the hand pump 3 with respect to the body 2. As a result, compressing the hand pump 3 can be efficiently repeated, and the display section 14 can be seen from the front side during the operation to readily confirm a blood pressure value.

It is to be noted that the structures of the pressure sensor, the control section, the display section, the body, the hand pump, the grasp section, restricting means, and others according to the present invention are not restricted to those exemplified in the foregoing embodiment, and they can be appropriately modified without departing from the scope of the invention.

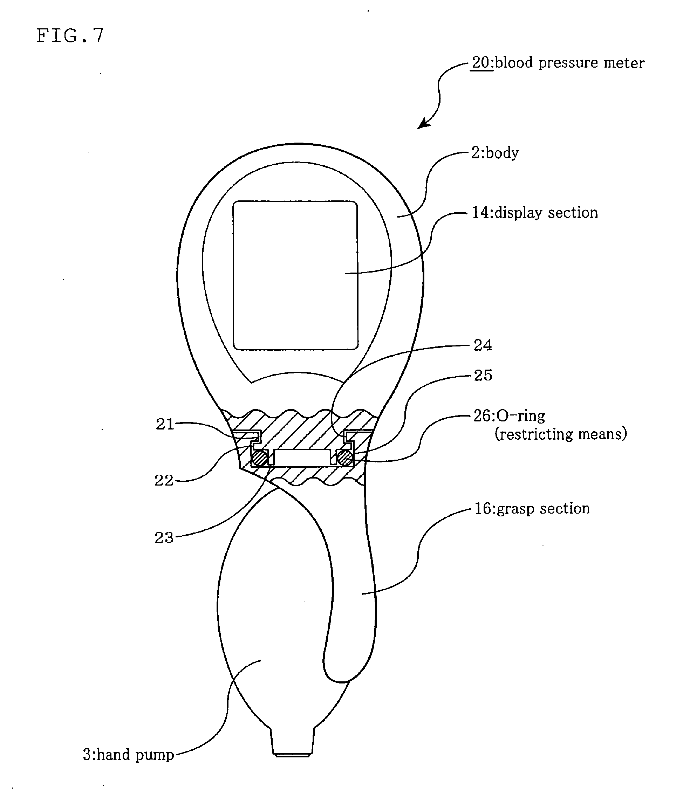

For example, although the example where the grasp section 16 is integrally arranged to the body 2 has been explained in the foregoing embodiment, a blood pressure meter 20 in which a grasp section 16 is arranged to be rotatable around an axis line of a hand pump 3 with respect to a body as shown in FIG. 7 may be adopted in place of the above configuration.

In this blood pressure meter 20, a small-diameter portion 21 and a large-diameter portion 22 are sequentially formed at a lower portion of the body 2 toward the lower side, and a cylindrical portion 23 are substantially concentrically formed on a lower surface of the large-diameter portion 22. Furthermore, a small-diameter hole portion 24 and a large-diameter hole portion 25 are sequentially formed at an upper portion of the grasp section 16 toward the lower side.

Moreover, when the small-diameter hole portion 24 and the large-diameter hole portion 25 of the grasp section 16 are respectively fitted on the small-diameter portion 21 and the large-diameter portion 22 of the body 2, the grasp section 16 can be supported to be rotatable around the axis line of the hand pump 3 with respect to the body 2. Additionally, an O-ring (restricting means) 26 is interposed between the cylindrical portion 23 of the body 2 and the large-diameter hole portion 25 of the grasp section 16. The O-ring 26 slides on an outer peripheral surface of the cylindrical portion 23 of the body 2 and an inner peripheral surface of the large-diameter hole portion 25 of the grasp section 16, and a sliding friction thereby functions with respect to the grasp section 16, thus positioning and restricting the grasp section 16 at a predetermined rotation angle.

Since the grasp section 16 is arranged to be rotatable around the axis line of the hand pump 3 with respect to the body 2 and the O-ring 26 which positions and restricts the grasp section 16 at the predetermined rotation angle is provided in this manner, the central line of the grasp section 16 in the width direction can be adjusted to an arbitrary angle in the range of not less than 10° to less than 80° around the axis line of the hand pump 3 with respect to the display section 14.

Further, even if an operator is a left-handed person, when the grasp section 16 is rotated to be grasped by his/her left hand, the central line of the grasp section 16 in the width direction can be readily arranged in the range of not less than 10° to less than 80° about the axis line of the hand pump 3 with respect to the display section 14.

It is to be noted that a stopper may be provided in place of the 0-ring 26 to position and restrict the grasp section 16 at a predetermined rotation angle.

### Industrial Applicability

As explained above, the present invention can be extensively applied to the blood pressure meter which operates the hand pump to measure a blood pressure.

## Claims

1. A blood pressure meter comprising:
a body which is connected through a tube with a cuff detachably disposed to an arm of a measurement target person, has a pressure sensor which measures a pressure in the cuff, has a control section which determines a blood pressure value based on a fluctuation in the measured value, and has a display section which displays the determined blood pressure value;
a spherical hand pump which is attached to the body and supplies air to the cuff by a compressing operation to inflate the cuff; and
a grasp section which is extended along an outer surface of the hand pump from the body in an axial direction of the hand pump, and configured to be grasped together with the hand pump,
the display section being arranged in the body so that the blood pressure value is visible from a direction substantially perpendicular to an axis line of the hand pump,
wherein a central line of the grasp section in a width direction is arranged in a range of not less than 10° to less than 80° around the axis line of the hand pump with respect to the display section.

2. The blood pressure meter according to claim 1, wherein the central line of the grasp section in the width direction is arranged at a position at 45° around the axis line of the hand pump with respect to the display section.

3. The blood pressure meter according to claim 1 or 2, wherein a width dimension of the grasp section is less than 90° around the axis of the hand pump.

4. The blood pressure meter according to any one of claims 1 to 3, wherein an extended end of the grasp section from the body is arranged at a position which exceeds 1/2 of a length of the hand pump in the axial direction and is less than 4/5 of the same.

5. The blood pressure meter according to claim 4, wherein the extended end of the grasp section from the body is arranged on a side where it is apart from the body beyond a maximum diameter of the hand pump.

6. The blood pressure meter according to claim 4 or 5, wherein the grasp section is arranged to form a gap between itself and an outer surface of the hand pump along an extending direction from the body, and the extended end is in contact with the hand pump.

7. The blood pressure meter according to any one of claims 1 to 6, wherein the grasp section is arranged to be rotatable around the axis line of the hand pump with respect to the body,
the blood pressure meter including restricting means for positioning and restricting the grasp section at a predetermined rotation angle.
